# EUROPÄISCHE PATENTSCHRIFT

(11) **EP 0 714 288 B1**
(45) Veröffentlichungstag und Bekanntmachung des Hinweises auf die Patenterteilung: **07.02.2001**
(21) Anmeldenummer: 94926186.1
(22) Anmeldetag: 11.08.1994
(51) Int. Cl.: A61K 7/50, A61K 7/06, C11D 3/382, C11D 3/384

(54) **DETERGENSGEMISCHE**
DETERGENT MIXTURES
MELANGES DETERSIFS

(30) Priorität: 20.08.1993 US 109790
(43) Veröffentlichungstag der Anmeldung: 05.06.1996
(73) Patentinhaber: Cognis Deutschland GmbH, 40589 Düsseldorf (DE)
(72) Erfinder: SALKA, Barry, Fair Lawn, NJ 07410 (US); HENSEN, Hermann, D-42781 Haan (DE); TESMANN, Holger, D-41363 Jüchen (DE); KAHRE, Jörg, D-40789 Monheim (DE)
(86) Internationale Anmeldenummer: EP9402686
(87) Internationale Veröffentlichungsnummer: WO9505802

(56) Entgegenhaltungen:
- WO-A-93/21896
- WO-A-94/04124
- WO-A-94/07458
- DE-A- 4 139 935
- DE-A- 4 229 922

## Beschreibung

### Gebiet der Erfindung

Die Erfindung betrifft Detergensgemische enthaltend Alkyl-und/oder Alkenyloligoglykoside, Proteinhydrolysate und monomere kationische Tenside, ein Verfahren zur Verbesserung der Sprungkraft von Haaren, bei dem man Detergensgemische, enthaltend Alkyl- und/oder Alkenyloligoglykoside, Proteinhydrolysate und gegebenfalls monomere kationische Tenside anwendet, Haarfestigungsmittel, die die letzteren Detergensgemische enthalten sowie deren Verwendung zur Herstellung von Haarfestigungsmitteln.

### Stand der Technik

Das gewaschene Haar läßt sich infolge der Entfettung und Entfernung aller Schmutzpartikel nur schwer und widerwillig frisieren. Erschwerend für die Gestaltung einer haltbaren Frisur und deren Erhaltung wirkt sich die elektrostatische Aufladung des Haares aus. Sie ist abhängig von der Qualität des Shampoos und der Höhe der umgebenden Luftfeuchtigkeit und macht sich insbesondere im Winter bei warmer und trockener Heizungsluft bemerkbar. Zur Verbesserung der Kämmbarkeit von gewaschenem und frottiertem Haar, zur Konditionierung sowie zur Stabilisierung gegen äußere Einflüsse werden daher Haarfestigungsmittel eingesetzt.

Handelsübliche flüssige Haarfestiger stellen Lösungen von Filmbildnern in Wasser und/oder Alkohol dar. Zusätzlich werden Weichmacher verwendet, um den Film auf dem Haar flexibler zu machen. Kationische Tenside verbessern die Kämmbarkeit des Haares und setzen die elektrostatische Aufladung herab. Der Zusatz von Parfümölen rundet das fertige Produkt ab, während Farbstoffe verzichtbar sind, aber aus ästhetischen Gründen häufig eingesetzt werden. Als Filmbildner kommen üblicherweise polymere Stoffe, wie beispielsweise Polyvinylpyrrolidon und/oder Polyvinylacetat in Frage. Im Hinblick auf die ständig steigende Bedeutung ökotoxikologischer Eigenschaften von oberflächenaktiven Mitteln, die mit der menschlichen Haut bzw. Haaren in Kontakt treten, besteht ein nachhaltiges Bedürfnis an Produkten, die nicht nur in Summe, sondern auch bezogen auf ihre Einzelstoffe besonders umweltverträglich sind.

Alkyloligoglykoside, insbesondere Alkyloligoglucoside stellen nichtionische Tenside dar, die infolge ihrer ausgezeichneten Detergenseigenschaften, ihres breiten Eigenschaftsprofils und ihrer hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in **Skin Care Forum, 1, (Okt. 1992),** D.Balzer und N.Ripke in **Seifen-Öle-Fette-Wachse 118,** **894 (1992)** und B.Brancq in **Seifen-Öle-Fette-Wachse 118, 905 (1992)** genannt werden sollen.

Auch die gemeinsame Verwendung von Alkyloligoglucosiden und kationischen polymeren Tensiden in Haarbehandlungsmitteln ist aus einer Reihe von Druckschriften bekannt. So wird in der **US 4 668 422** (Henkel) eine Rezeptur für ein Schaumbad offenbart, die C_{9/11}- und C_{12/13}-Alkyloligoglucoside, Betaine, Aminoxide, Perlglanzmittel und kationische Copolymere von Acrylamid und Dimethyldiallylammoniumchlorid enthalten. Aus der **JP-A 01/144 497** (Shiseido) sind Haarshampoos bekannt, die neben C_{8/20}-Alkyloligoglykosiden, kationische Polymere, quartäre Ammoniumverbindungen und anionische Tenside enthalten. In der **DE-A 30 18 600** (L'Oreal) wird in Beispiel 16 ein Haarshampoo vorgeschlagen, das neben C_{8/20}-Alkyloligoglucosiden und Saponinen kationische Stärke enthält. Gegenstand der **EP-A1 0 337 354** sind schließlich milde Tensidmischungen enthaltend Alkyloligoglucoside und kationische Polymere.

Die **WO 93/21896** beschreibt ein Verfahren zur Dauerhaften Verformung von Haaren, bei dem man wäßrige Zubereitungen aus Alkylglykosiden und anionischen oder kationischen Tensiden und/oder einer polymere Verbindungen einsetzt.

Die **DE 4139935 A1** offenbart ein flüssiges Körperreinigungsmittel aus einem anionischem Tensid, einem Alkylpolyglykosid und einem C₈-C₁₈ Alkylmonoglycerid.

Keine dieser Druckschriften offenbart jedoch den Einsatz von Alkyloligoglucosiden in Haarfestigungsmitteln.

Die gemeinsame Verwendung von Alkyloligoglykosiden und Proteinhydrolysaten ist grundsätzlich ebenfalls bekannt. So werden beispielsweise in **EP-A 0 308 189, EP-A-0 308 190** und **EP-A 0 309 259** Toilettenstückseifen beschrieben, die als Tensidkomponente u. a. Alkyloligoglykoside und als Feuchtigkeitsmittel Proteinhydrolysate enthalten können. Ein Bezug zur Haarkosmetik besteht jedoch auch hier nicht.

Die Aufgabe der Erfindung bestand somit darin, neue Haasfestigungsmittel mit gut dermatologisch verträglichen und leicht biologisch abbaubaren Filmbildnern zu entwickeln. Eine weitere Aufgabenstellung hat ferner darin bestanden, Produkte zur Verfügung zu stellen, die eine verbesserte Ausspülbarkeit bzw. Ausbürstbarkeit aufweisen.

### Beschreibung der Erfindung

Gegenstand der Erfindung sind Detergensgemische, enthaltend
a) Alkyl- und/oder Alkenyloligoglykoside der Formel **(I)**

   **R**^{**1**}**O-[G]**_{**p**} **(I)**

   in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 1000 bis 10.000 und
c) monomere kationische Tenside.

Überraschenderweise wurde gefunden, daß Detergensgemische enthaltend Alkyl- und/oder Alkenyloligoglykoside, Proteinhydrolysate und gegebenenfalls kationische Tenside in der Lage sind, die Sprungkraft von Haaren in einer Weise zu verbessern, wie dies durch konventionelle Mischungen von Polymeren und Kationtensiden bislang nicht möglich gewesen ist. Die Produkte lassen sich leicht auswaschen, leicht ausbürsten und zeichnen sich durch eine ausgezeichnete dermatologische und ökologische Verträglichkeit aus. Die Erfindung schließt dabei insbesondere auch die Erkenntnis ein, daß Mischungen von Alkyl- und/oder Alkenyloligoglykosiden und Proteinhydrolysaten als Filmbildner wirken können, eine Eigenschaft, die ansonsten nur von hochpolymeren Systemen bekannt ist.

### Alkyl- und/oder Alkenyloligoglykoside

Alkyl- und Alkenyloligoglykoside stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/ oder Alkenyloligoglykoside sind somit Alkyl- und/oder Alkenyloligo**glucoside.**

Die Indexzahl p in der allgemeinen Formel **(I)** gibt den Oligomerisierungsgrad (DP-Grad), d. h. die Verteilung von Mono-und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkylund/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 6 bis 22, vorzugsweise 8 bis 16 Kohlenstoffatomen ableiten. Typische Beispiele sind Capronalkohol, Caprylalkohol, Caprinalkohol, Undecylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside auf Basis von gehärtetem C_{8/16}-Kokosalkohol mit einem DP von 1 bis 3.

### Proteinhydrolysate

Proteinhydrolysate stellen Stoffe dar, die man üblicherweise durch Abbau von tierischem Eiweiß, vorzugsweise Rinderkollagen herstellt. Bei der **alkalischen Hydrolyse** erfolgt eine unspezifische Öffnung der Peptidbindungen, die den Regeln der Statistik folgt. Da die Carboxygruppen der Peptide während der Hydrolyse als Salz vorliegen, während die Aminogruppen ungeschützt sind und teilweise abgespalten werden können, resultiert ein Hydrolysat, bei dem die Polypeptide eine höhere Zahl von Carboxy- als Aminogruppen aufweisen. Auch die **saure Hydrolyse** führt zu einer unspezifischen Öffnung der Peptidbindungen. Anders als bei der alkalischen Hydrolyse, liegen beim sauren Abbau jedoch die Aminogruppen als Salz und die Carboxygruppen in freier Form vor, die jedoch eine wesentlich höhere Stabilität als die ungeschützten Aminogruppen aufweisen. Die Produkte weisen somit in Abhängigkeit des Hydrolyseverfahrens starke Strukturunterschiede auf [vgl. **J.Am. Oil.Chem.Soc., 59, 217 (1982); Seifen-Öle-Fette-Wachse 108, 177 (1982)].**

Vorzugsweise finden im Sinne der Erfindung solche Proteinhydrolysate Verwendung, die auf **enzymatischem Wege** hergestellt worden sind. Hierbei läßt man spezifisch auf die Peptidbindung wirkende Enzyme angreifen. Die Produkte weisen eine hohe Homogenität sowie insbesondere eine gegenüber den übrigen Hydrolysaten verbesserte dermatologische Verträglichkeit auf. Das durchschnittliche Molgewicht kann über die Reaktionsbedingungen eingestellt werden und - wie bei den alkalischen bzw. sauren Hydrolysaten auch - im Bereich von 1.000 bis 10.000, vorzugsweise 2.000 bis 6.000 liegen.

Neben tierischem Eiweiß werden vorzugsweise Proteinhydrolysate eingesetzt, die durch enzymatischen Abbau von pflanzlichem Eiweiß gewonnen werden. Typische Beispiele sind Hydrolysate von Sojaprotein, Mandelprotein, Weizenprotein und/oder Kartoffelprotein. Gegenüber tierischen Produkten haben die genannten Proteinhydrolysate ebenfalls wieder den Vorteil einer verbesserten dermatologischen Verträglichkeit.

Die Detergensgemische können die Alkyl- und/oder Alkenyloligoglykoside und die Proteinhydrolysate im Gewichtsverhältnis 1 : 5 bis 5 : 1, vorzugsweise 1 : 3 bis 3 : 1 - bezogen auf die Gemische - enthalten.

### Monomere kationische Tenside

Als monomere kationische Tenside kommen beispielsweise quartäre Ammoniumverbindungen (QAV) der Formel **(II)** in Betracht, 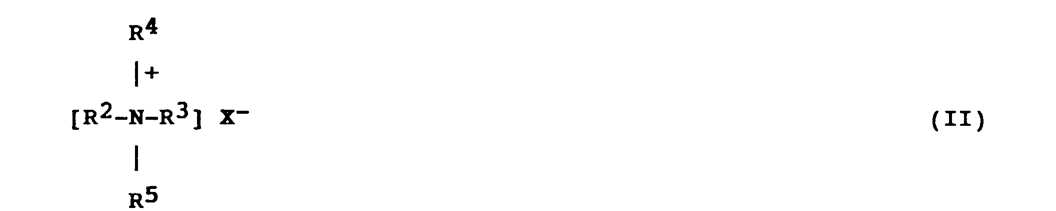 in der R² für einen gegebenenfalls hydroxysubstituierten Alkyl- und/oder Alkenylrest mit 6 bis 22 Kohlenstoffatomen, R³ für R² oder einen Alkylrest mit 1 bis 5 Kohlenstoffatomen und R⁴ und R⁵ unabhängig voneinander für Alkylreste mit 1 bis 5 Kohlenstoffatomen sowie X für Halogen, Phosphat, Alkylsulfat oder Alkylphosphat steht.

Bei den QAV handelt es sich um bekannte Stoffe, die nach den einschlägigen Verfahren der präparativen organischen Chemie erhalten werden können. Eine Methode zu ihrer Herstellung besteht beispielsweise darin, tertiäre Amine mit Methylchlorid oder Dimethylsulfat zu quaternieren.

Typische Beispiele für im Sinne der Erfindung geeignete QAV stellen Stearyltrimethylammoniumchlorid, Distearyldimethylammoniumchlorid, Cetyltrimethylammoniumchlorid (Dehyquart^{(R)} A, Henkel), Dicetyldimethylammoniumchlorid, 2-Hydroxycetyl-2-hydroxyethyldimethylammoniumchlorid (Dehyquart^{(R)} E, Henkel) oder Oxyethylammoniumphosphat (Dehyquart^{(R)} SP, Henkel) dar. Vorzugsweise werden solche QAV eingesetzt, in denen R² für einen gegebenenfalls hydroxysubstituierten Alkylrest mit 16 bis 18 Kohlenstoffatomen, R³ für R², eine Methyl- oder Hydroxyethylgruppe, R⁴ und R⁵ für eine Methylgruppe und X für Chlorid oder Methylsulfat steht.

Eine weitere Gruppe im Sinne der Erfindung geeigneter monomerer kationischer Tenside, stellen die **Esterquats** der Formel **(III)** dar, 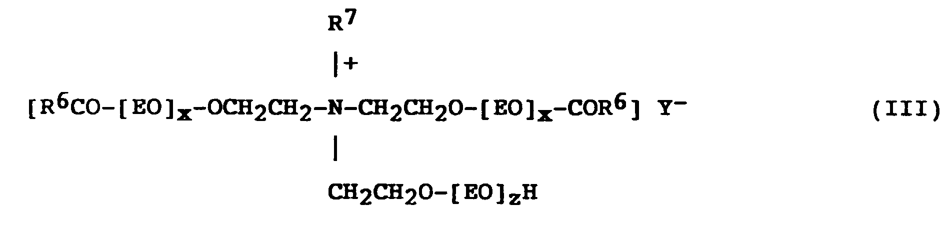 in der R⁶CO für einen aliphatischen, gegebenenfalls hydroxysubstituierten Acylrest mit 12 bis 22 Kohlenstoffatomen und 0 oder 1 Doppelbindung, R⁷ für eine Methylgruppe oder eine Polyethylenglycoletherkette mit 1 bis 5 Ethylenoxideinheiten, x, y und z für 0 oder in Summe für Zahlen von 1 bis 5 und Y für Halogen, Alkylsulfat oder Alkylphosphat steht.

Esterquats stellen technische Gemische von gegebenenfalls ethoxylierten Mono- und Difettsäure-triethanolaminestern dar, die mit Ethylenoxid, Alkylhalogeniden, Dialkylsulfaten oder Dialkylphosphaten quaterniert sind. Auch bei ihnen handelt es sich um bekannte Stoffe.

Vorzugsweise werden Esterquats eingesetzt, die sich von Talg-Kokos- und/oder Palmfettsäuren einer Iodzahl im Bereich 0 bis 40 ableiten und einen Veresterungsgrad von 1,5 bis 1,9 aufweisen. Der Ethoxylierungsgrad kann 0 oder 1 bis 5, vorzugsweise 1 bis 3 betragen. Aus anwendungstechnischen Gründen besonders geeignet sind Esterquats der Formel **(I)**, in der R⁷ für eine Methylgruppe oder eine Polyethylenglycolkette mit 1 bis 3 Ethy- lenoxideinheiten und Y für Chlorid oder Methylsulfat steht. Besonders bevorzugt ist ein methylquaternierter Dipalmfettsäuretriethanolaminester (Dehyquart^{(R)} AU36, Pulcra/ Barcelona).

Die Alkyl- und/oder Alkenyloligoglykoside und die monomeren kationischen Tenside können in den Detergensgemischen im Gewichtsverhältnis 1 : 10 bis 10 : 1, vorzugsweise 1 : 1 bis 7 : 1 - bezogen auf die Gemische - enthalten sein.

Die erfindungsgemäßen Detergensgemische können durch einfaches Verrühren, gebenenenfalls bei leicht erhöhter Temperatur (30 bis 40°C), hergestellt werden. Hierbei handelt es sich um einen rein mechanischen Vorgang, eine chemische Reaktion findet nicht statt. Zur Herstellung können wasserfreie oder hochkonzentrierte Ausgangsstoffe verwendet werden, die anschließend mit Wasser auf die gewünschte Anwendungskonzentration verdünnt werden. Es jedoch ebenso möglich, verdünnte Lösungen zu mischen.

Ein weiterer Gegenstand der Erfindung betrifft ein Verfahren zur Festigung von Haaren, bei dem man Haare in an sich bekannter Weise mit Detergensmischungen, enthaltend
a) Alkyl- und/oder Alkenyloligoglykoside der Formel **(I)**

   **R**^{**1**}**O-[G]**_{**p**} **(I)**

   in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 500 bis 5000 und gegebenenfalls
c) monomere kationische Tenside,
behandelt.

Vorteilhafterweise wird die Detergensmischung, abhängig von der Haarfülle und der Haarlänge, in einer Menge von beispielsweise 15 bis 25 ml auf das gewaschene und frottierte Haar aufgetragen und gleichmäßig verteilt. Dabei ist es zweckmäßig, das Haar durchzukämmen, um eine gleichmäßige Verteilung des Haarfestigers sicherzustellen. Anschließend wird das Haar auf Wasserwellwickler aufgerollt und an der Luft, vorzugsweise unter einer Trockenhaube getrocknet. Es ist jedoch ebenfalls möglich, auf das Aufrollen zu verzichten und das Haar durch eine Fönbürste zu formen und gleichzeitig zu trocknen ("Fönfestigen").

Ein weiterer Gegenstand der Erfindung betrifft Haarfestigungsmittel, enthaltend
a) Alkyl- und/oder Alkenyloligoglykoside der Formel **(I)**

   **R**^{**1**}**O-[G]**_{**p**} **(I)**

   in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 500 bis 5000 und gegebenenfalls
c) monomere kationische Tenside.

Die Haarfestigungsmittel kommen vorzugsweise als flüssige wäßrige und/oder alkoholische, insbesondere ethanolische Lösungen mit einem Aktivsubstanzgehalt von ca. 5 bis 30, vorzugsweise 10 bis 20 Gew.-% - bezogen auf die Lösung - in Betracht. Als weitere Zusatzstoffe können sie beispielsweise filmbildende Polymere, wie beispielsweise Polyvinylpyrrolidon und/oder Polyvinylacetat, Farbstoffe, Parfümstoffe, UV-Adsorber, Verdickungsmittel enthalten. Neben der flüssigen Anbietungsform können die erfindungsgemäßen Mittel auch als Schäume eingesetzt werden ("Schaumfestiger").

### Gewerbliche Anwendbarkeit

Detergensgemische enthaltend Alkyl- und/oder Alkenyloligoglykoside, Proteinhydrolysate und gegebenenfalls monomere kationische Tenside zeigen filmbildende und konditionierende Eigenschaften. Sie verleihen dem Haar Halt, Fülle, Frisierbarkeit, Festigkeit und Sprungkraft.

Ein weiterer Gegenstand der Erfindung betrifft daher die Verwendung von Detergensgemischen, enthaltend
a) Alkyl- und/oder Alkenyloligoglykoside der Formel **(I)**

   **R**^{**1**}**O-[G]**_{**p**} **(I)**

   in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 1.000 bis 10.000 und gegebenenfalls
c) monomere kationische Tenside
zur Herstellung von Haarfestigungsmitteln, in denen sie in Mengen von 10 bis 95, vorzugsweise 30 bis 80 Gew.-% - bezogen auf die Mittel - enthalten sein können.

Die folgenden Beispiele sollen den Gegenstand der Erfindung näher erläutern, ohne ihn darauf einzuschränken.

### Beispiele

### I. Eingesetzte Stoffe

A) Plantaren^{(R)} 2000, Fa.Henkel KGaA, Düsseldorf/FRG C_{8/16}-Alkyloligoglucosid (DP = 1,4)
B) Nutrilan^{(R)} I, Fa.CF Grünau, Illertissen/FRG Kollagenhydrolysat, Mittleres Molgewicht ca. 4800
C) Ethylalkohol, kosmetisch, 96 Gew.-%ig
D) Dehyquart^{(R)} SP, Fa.Henkel KGaA, Düsseldorf/FRG Oxyethylammoniumphosphat

### II. Rezepturen

**Tab.1:**

| Eingesetzte Rezepturen | | | | |
|---|---|---|---|---|
| Rezeptur | Komponenten (Gew.-%)* | | | |
| | A | B | C | D |
| A | 6,0 | 3,8 | 30,0 | 1,0 |
| B | 6,0 | - | 30,0 | 1,0 |

| | | | | |
|---|---|---|---|---|
| *) ad 100 dest.Wasser | | | | |

### III. Anwendungstechnische Beispiele

Zur Messung der Sprungkraft wurden Haarsträhnen vom Typ "European dark" code 6634 der Fa.Alkinco (12 cm lang, ca. 1 g schwer) mit 250 mg Wasser bzw. Festiger gemäß Rezeptur A bzw. B pro Strähne behandelt. Die Applikation erfolgte auf dem feuchten Haar. Die Rezeptur A war erfindungsgemäß, die Rezeptur B diente dem Vergleich; in beiden Fällen wurden die Festiger auf pH = 6,6 eingestellt. Nach der Behandlung wurden die Strähnen ohne Ausspülen auf Lockenwickler (Durchmesser 48 mm) aufgebracht und 2 h bei 40°C getrocknet. Bestimmt wurde das Schwingungsverhalten der Locken im nicht- bzw. gekämmten Zustand bei 15 % relativer Luftfeuchte. Die Mittelwerte der 10-fach Bestimmung hinsichtlich Frequenz, Dämpfung, 0-Amplitude, Streckkraft und Streckarbeit sind in den Tab.2 und 3 zusammengefaßt.

**Tab.2:**

| Erfindungsgemäße Beispiele | | | | | | | |
|---|---|---|---|---|---|---|---|
| Bsp. | F s⁻¹ | Dämpfung | | 0-Amplitude | | StK mN | StA mJ |
| | | Min. s⁻¹ | Max. s⁻¹ | Min. mN | Max. mN | | |
| 1a | 2,86 | 0,89 | 0,97 | 3,88 | 3,78 | 11,01 | 0,46 |
| 1b | 4,33 | 1,81 | 1,98 | 7,91 | 8,89 | 31,77 | 1,02 |
| 1c | 2,96 | 1,22 | 1,18 | 4,19 | 3,94 | 11,12 | 0,43 |
| 1d | 4,19 | 1,63 | 1,91 | 9,26 | 8,81 | 31,56 | 0,92 |
| Legende: F = Frequenz StK = Streckkraft StA = Streckarbeit Min = Minimum Max = Maximum a) Wasserwelle, Locken nicht ausgekämmt b) Testrezeptur A, Locken nicht ausgekämmt c) Wasserwelle, Locken ausgekämmt d) Testrezeptur A, Locken ausgekämmt | | | | | | | |

**Tab.3:**

| Vergleichsversuche | | | | | | | |
|---|---|---|---|---|---|---|---|
| Bsp. | F s⁻¹ | Dämpfung | | 0-Amplitude | | StK mN | StA mJ |
| | | Min. s⁻¹ | Max. s⁻¹ | Min. mN | Max. mN | | |
| V1a | 2,72 | 0,08 | 0,90 | 4,17 | 3,43 | 9,12 | 0,33 |
| V1b | 4,29 | 2,14 | 2,45 | 6,90 | 7,96 | 39,20 | 1,33 |
| V1c | 2,91 | 1,21 | 1,29 | 5,09 | 4,62 | 10,16 | 0,35 |
| V1d | 3,23 | 1,29 | 1,47 | 4,42 | 4,62 | 21,04 | 0,79 |
| Legende: a) Wasserwelle, Locken nicht ausgekämmt b) Testrezeptur B, Locken nicht ausgekämmt c) Wasserwelle, Locken ausgekämmt d) Testrezeptur B, Locken ausgekämmt | | | | | | | |

Die Beispiele und Vergleichsbeispiele zeigen, daß Rezepturen, die als nichtionische Tenside Alkyloligoglucoside und Proteinhydroysate enthalten, im Vergleich zur bloßen Wasserbehandlung (Wasserwelle) sowie Rezepturen, die als nichtionische Tenside Alkyloligoglucoside allein enthalten, eine Verbesserung der Sprungkraft, ausgedrückt durch höhere Streckarbeiten, Schwingungsfrequenzen und Anfangsamplituden bewirken. Zusammen mit der verstärkten Schwingung wird auch eine vermehrte Reibung im Haarverband und damit auch eine erhöhte Dämpfung beobachtet. Dabei macht es keinen Unterschied, ob nicht ausgekämmte Locken oder ausgekämmte Locken betrachtet werden.

Bei ersteren ist der Effekt besonders deutlich, da der stark verklebte Strähnenverband zum Schwingen gebracht werden muß.

In Tab.4 ist das Schwingungsverhalten von Locken dargestellt. Die Meßwerte stellen die Dämpfung der Maxima, die 0-Amplitude der Maxima sowie die Streckarbeit der ausgekämmten Locken bei 15 % relativer Luftfeuchtigkeit dar.

**Tab.4:**

| Schwingungsverhalten von Locken | | | | |
|---|---|---|---|---|
| Bsp. | R | Dämpfung s⁻¹ | 0-Amplitude mN | Streckarbeit mJ |
| 2 | A | 1,90 | 8,90 | 0,93 |
| V2 | B | 1,45 | 4,75 | 0,79 |
| Legende: R = Rezeptur | | | | |

## Patentansprüche

1. Detergensgemische, enthaltend
a) Alkyl- und/oder Alkenyloligoglykoside der Formel **(I)**
**R**^{**1**}**O-[G]**_{**p**} **(I)**
in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 1.000 bis 10.000 und
c) monomere kationische Tenside.

2. Detergensgemische nach Anspruch **1, dadurch gekennzeichnet,** daß sie Alkyloligoglucoside der Formel **(I)** enthalten, in der R¹ für einen Alkylrest mit 8 bis 16 Kohlenstoffatomen, G für einen Glucoserest und p für Zahlen von 1 bis 3 steht.

3. Detergensgemische nach den Ansprüchen 1 und 2, **dadurch gekennzeichnet,** daß sie Proteinhydrolysate enthalten, die ein durchschnittliches Molekulargewicht im Bereich von 2000 bis 6000 aufweisen.

4. Detergensgemische nach den Ansprüchen 1 bis 3, **dadurch gekennzeichnet,** daß sie Proteinhydrolysate enthalten, die durch enzymatischen Abbau von pflanzlichen Proteinen hergestellt werden.

5. Detergensgemische nach den Ansprüchen 1 bis 4, **dadurch gekennzeichnet,** daß sie als monomere kationische Tenside quartäre Ammoniumverbindungen der Formel **(II)** enthalten, 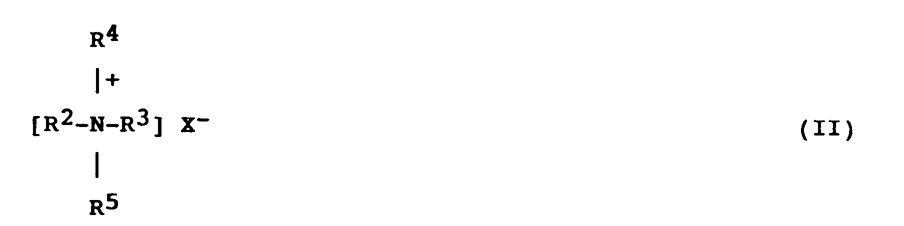 in der R² für einen gegebenenfalls hydroxysubstituierten Alkyl- und/oder Alkenylrest mit 6 bis 22 Kohlenstoffatomen, R³ für R² oder einen Alkylrest mit 1 bis 5 Kohlenstoffatomen und R⁴ und R⁵ unabhängig voneinander für Alkylreste mit 1 bis 5 Kohlenstoffatomen sowie X für Halogen, Alkylsulfat oder Alkylphosphat steht.

6. Detergensgemische nach den Ansprüchen 1 bis 4, **dadurch gekennzeichnet,** daß sie als monomere kationische Tenside Esterquats der Formel **(III)** enthalten, 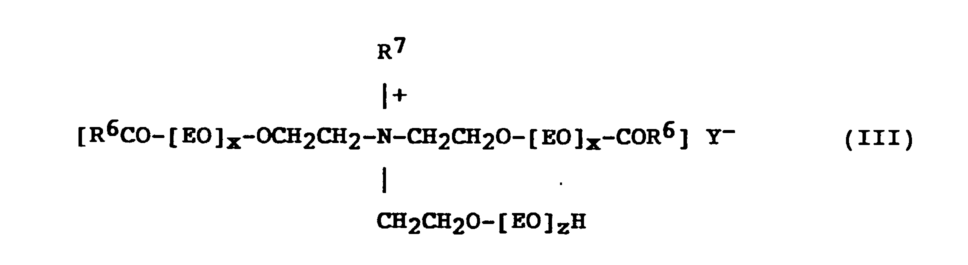 in der R⁶CO für einen aliphatischen, gegebenenfalls hydroxysubstituierten Acylrest mit 12 bis 22 Kohlenstoffatomen mit 0 oder 1 Doppelbindung, R⁷ für eine Methylgruppe oder eine Polyethylenglycoletherkette mit 1 bis 5 Ethylenoxideinheiten, x, y und z für 0 oder in Summe für Zahlen von 1 bis 5 und Y für Halogen, Alkylsulfat oder Alkylphosphat steht.

7. Detergensgemische nach den Ansprüchen 1 bis 6, **dadurch gekennzeichnet,** daß sie die Alkyl- und/oder Alkenyloligoglykoside und die Proteinhydrolysate im Gewichtsverhältnis 1 : 5 bis 5 : 1 - bezogen auf die Gemische - enthalten.

8. Detergensgemische nach den Ansprüchen 1 bis 7, **dadurch gekennzeichnet,** daß sie die Alkyl- und/oder Alkenyloligoglykoside und die monomeren kationischen Tenside im Gewichtsverhältnis 1 : 10 bis 10 : 1 - bezogen auf die Gemische - enthalten.

9. Verfahren zur Festigung von Haaren, bei dem man Haare in an sich bekannter Weise mit Detergensmischungen, enthaltend
a) Alkyl- und/oder Alkenyloligoglykoside der Formel **(I)**
**R**^{**1**}**O-[G]**_{**p**} **(I)**
in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 1000 bis 10.000 und gegebenenfalls
c) monomere kationische Tenside,
behandelt.

10. Haarfestigungsmittel, enthaltend
a) Alkyl- und/oder Alkenyloligoglykoside der Formel **(I)**
**R**^{**1**}**O-[G]**_{**p**} **(I)**
in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 2000 bis 6000 und gegebenenfalls
c) monomere kationische Tenside.

11. Verwendung von Detergensgemischen, enthaltend
a) Alkyl- und/oder Alkenyloligoglykoside der Formel **(I)**
**R**^{**1**}**O-[G]**_{**p**} **(I)**
in der R¹ für einen Alkyl- und/oder Alkenylrest mit 6 bis 22 Kohlenstoffatomen, G für einen Zuckerrest mit 5 oder 6 Kohlenstoffatomen und p für Zahlen von 1 bis 10 steht,
b) Proteinhydrolysate mit einem durchschnittlichen Molekulargewicht im Bereich von 1000 bis 10.000 und gegebenenfalls
c) monomere kationische Tenside
zur Herstellung von Haarfestigungsmitteln.

## Claims

1. Detergent mixtures containing
a) alkyl and/or alkenyl oligoglycosides corresponding to formula (I)
R¹O-[G]ₚ (I)
in which R¹ is an alkyl and/or alkenyl radical containing 6 to 22 carbon atoms, G is a sugar unit containing 5 or 6 carbon atoms and p is a number of 1 to 10,
b) protein hydrolyzates having an average molecular weight in the range from 1,000 to 10,000 and
c) monomeric cationic surfactants.

2. Detergent mixtures as claimed in claim 1, characterized in that they contain alkyl oligoglucosides corresponding to formula (I), in which R¹ is an alkyl radical containing 8 to 16 carbon atoms, G is a glucose unit and p is a number of 1 to 3.

3. Detergent mixtures as claimed in claims 1 and 2, characterized in that they contain protein hydrolyzates which have an average molecular weight in the range from 2,000 to 6,000.

4. Detergent mixtures as claimed in claims 1 to 3, characterized in that they contain protein hydrolyzates produced by enzymatic degradation of vegetable proteins.

5. Detergent mixtures as claimed in claims 1 to 4, characterized in that they contain quaternary ammonium compounds corresponding to formula (II): 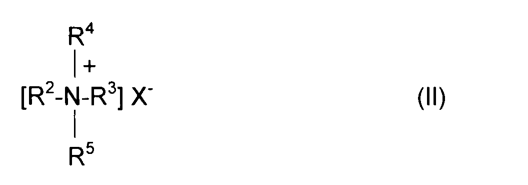 in which R² is an optionally hydroxy-substituted alkyl and/or alkenyl radical containing 6 to 22 carbon atoms, R³ has the same meaning as R² or is an alkyl radical containing 1 to 5 carbon atoms, R⁴ and R⁵ independently of one another represent alkyl radicals containing 1 to 5 carbon atoms and X represents halogen, alkyl sulfate or alkyl phosphate,
as monomeric cationic surfactants.

6. Detergent mixtures as claimed in claims 1 to 4, characterized in that they contain esterquats corresponding to formula (III): 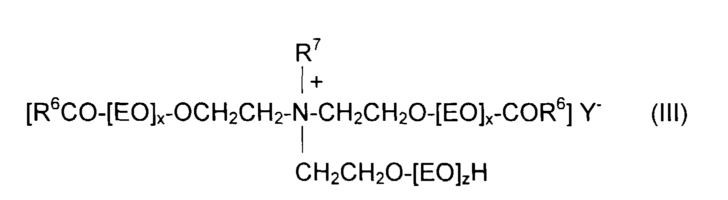 in which R⁶CO represents an aliphatic, optionally hydroxy-substituted acyl radical containing 12 to 22 carbon atoms and 0 or 1 double bond, R⁷ is a methyl group or a polyethylene glycol ether chain containing 1 to 5 ethylene oxide units, x, y and z = 0 or, together, stand for numbers of 1 to 5 and Y represents halogen, alkyl sulfate or alkyl phosphate,
as monomeric cationic surfactants.

7. Detergent mixtures as claimed in claims 1 to 6, characterized in that they contain the alkyl and/or alkenyl oligoglycosides and the protein hydrolyzates in a ratio by weight of 1:5 to 5:1, based on the mixture.

8. Detergent mixtures as claimed in claims 1 to 7, characterized in that they contain the alkyl and/or alkenyl oligoglycosides and the monomeric cationic surfactants in a ratio by weight of 1:10 to 10:1, based on the mixtures.

9. A process for setting hair, in which hair is treated in known manner with detergent mixtures containing
a) alkyl and/or alkenyl oligoglycosides corresponding to formula (I):
R¹O-[G]ₚ (I)
in which R¹ is an alkyl and/or alkenyl radical containing 6 to 22 carbon atoms, G is a sugar unit containing 5 or 6 carbon atoms and p is a number of 1 to 10,
b) protein hydrolyzates having an average molecular weight in the range from 1,000 to 10,000 and
c) monomeric cationic surfactants.

10. Hair setting preparations containing
a) alkyl and/or alkenyl oligoglycosides corresponding to formula (I):
R¹O-[G]ₚ (I)
in which R¹ is an alkyl and/or alkenyl radical containing 6 to 22 carbon atoms, G is a sugar unit containing 5 or 6 carbon atoms and p is a number of 1 to 10,
b) protein hydrolyzates having an average molecular weight in the range from 2,000 to 6,000 and
c) monomeric cationic surfactants.

11. The use of detergent mixtures containing
a) alkyl and/or alkenyl oligoglycosides corresponding to formula (I):
R¹O-[G]ₚ (I)
in which R¹ is an alkyl and/or alkenyl radical containing 6 to 22 carbon atoms, G is a sugar unit containing 5 or 6 carbon atoms and p is a number of 1 to 10,
b) protein hydrolyzates having an average molecular weight in the range from 1,000 to 10,000 and
c) monomeric cationic surfactants
for the production of hair setting preparations.

## Revendications

1. Mélanges de détergents contenant :
a) des alkyl- et/ou alkényloligoglycosides de formule (I)
R¹O-[G]ₚ (I)
dans laquelle R¹ représente un radical alkyle et/ou alkényle, ayant de 6 à 22 atomes de carbone, G représente un reste de sucre ayant 5 ou 6 atomes de carbone et p représente des nombres allant de 1 à 10,
b) des hydrolysats de protéine ayant un poids moléculaire moyen dans la zone de 1000 à 10.000 et,
c) des agents tensioactifs cationiques monomères.

2. Mélanges de détergents selon la revendication 1,
caractérisés en ce qu'
ils contiennent des alkyoligoglucosides de formule (I) dans lesquels R¹ représente un radical alkyle ayant de 8 à 16 atomes de carbone, G représente un reste de glucose et p représente des nombres allant de 1 à 3.

3. Mélanges de détergents selon les revendications 1 et 2,
caractérisés en ce qu'
ils contiennent des hydrolysats de protéine qui possèdent un poids moléculaire moyen dans la zone de 2000 à 6000.

4. Mélanges de détergents selon les revendications 1 à 3,
caractérisés en ce qu'
ils contiennent des hydrolysats de protéine qui sont produits par dégradation enzymatique de protéines végétales.

5. Mélanges de détergents selon les revendications 1 à 4,
caractérisés en ce qu'
ils contiennent comme agents tensioactifs cationiques monomères, des composés d'ammonium quaternaires de formule (II) 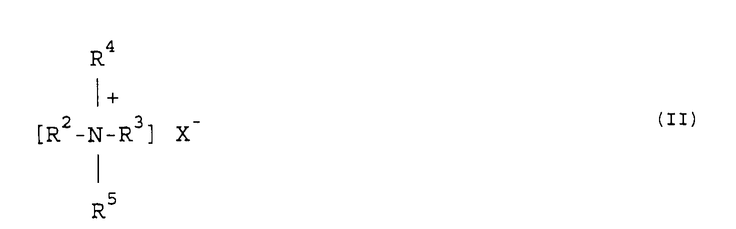 dans laquelle R² représente un radical alkyle et/ou alkenyle éventuellement substitué par un hydroxy ayant de 6 à 22 atomes de carbone, R³ représente R² ou un radical alkyle ayant de 1 à 5 atomes de carbone et R⁴ et R⁵ indépendamment l'un de l'autre représentent des radicaux alkyl ayant de 1 à 5 atomes de carbone ainsi que X représente un halogène, un alkylsulfate ou un alkylphosphate.

6. Mélanges de détergents selon les revendications 1 à 4,
caractérisés en ce qu'
ils contiennent comme agents tensioactifs cationiques monomères, des esterquats de formule (III), 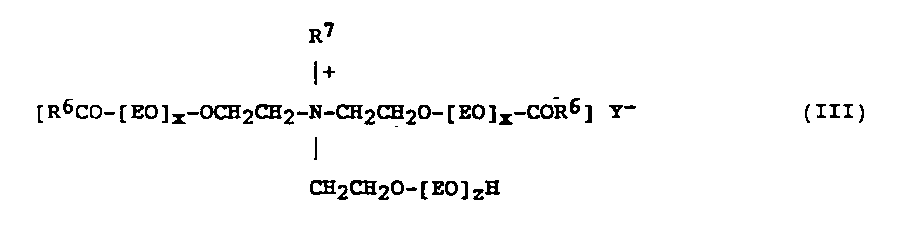 dans laquelle R⁶CO représente un reste acyle aliphatique, éventuellement substitué par un hydroxy ayant de 12 à 22 atomes de carbone et 0 ou 1 double liaison, R⁷ représente un radical méthyle ou une chaîne éther de polyéthylèneglycol ayant de 1 à 5 éléments oxyde d'éthylène, x, y et z représentent 0 ou globalement des nombres allant de 1 à 5, et Y représente un halogène, un alkylsulfate ou un alkylphosphate.

7. Mélanges de détergents selon les revendications 1 à 6,
caractérisés en ce qu'
ils contiennent les alkyl- et/ou alkényloligoglycosides et les hydrolysats de protéine dans un rapport en poids de 1 : 5 à 5 : 1, rapporté aux mélanges.

8. Mélanges de détergents selon les revendications 1 à 7,
caractérisés en ce qu'
ils contiennent les alkyl- et/ou alkényloligoglycosides et les agents tensioactifs cationiques monomères dans un rapport en poids de 1 : 10 à 10 : 1, rapporté aux mélanges.

9. Procédé de fixation des cheveux dans lequel on traite les cheveux d'une manière connue en soi avec des mélanges de détergents qui contiennent :
a) des alkyl- et/ou alkényloligoglycosides de formule (I)
R¹O-[G]ₚ (I)
dans laquelle R¹ représente un radical alkyle et/ou alkenyle, ayant de 6 à 22 atomes de carbone, G représente un reste de sucre ayant 5 ou 6 atomes de carbone et p représente des nombres allant de 1 à 10,
b) des hydrolysats de protéine ayant un poids moléculaire moyen dans la zone de 1000 à 10.000 et, éventuellement
c) des agents tensioactifs cationiques monomères.

10. Agent de fixation des cheveux, contenant :
a) des alkyl- et/ou alkényloligoglycosides de formule (I)
R¹O-[G]ₚ (I)
dans laquelle R¹ représente un radical alkyle et/ou alkényle, ayant de 6 à 22 atomes de carbone, G représente un reste de sucre ayant 5 ou 6 atomes de carbone et p représente des nombres allant de 1 à 10,
b) des hydrolysats de protéine ayant un poids moléculaire moyen dans la zone de 2 000 à 6 000 et, éventuellement
c) des agents tensioactifs cationiques monomères.

11. Utilisation de mélanges de détergents contenant :
a) des alkyl- et/ou alkényloligoglycosides de formule (I)
R¹O-[G]ₚ (I)
dans laquelle R¹ représente un radical alkyle et/ou alkényle, ayant de 6 à 22 atomes de carbone, G représente un reste de sucre ayant 5 ou 6 atomes de carbone et p représente des nombres allant de 1 à 10,
b) des hydrolysats de protéine ayant un poids moléculaire moyen dans la zone de 1000 à 10.000 et, éventuellement
c) des agents tensioactifs cationiques monomères pour la production d'agents de fixation des cheveux.
